# EUROPEAN PATENT APPLICATION

(11) **EP 2 133 432 A1**
(43) Date of publication of application: **16.12.2009**
(21) Application number: 08718478.4
(22) Date of filing: 07.03.2008
(51) Int. Cl.: C12Q 1/68, G01N 33/50, A61P 19/00

(54) **USE OF SNAIL1 ACTIVITY INHIBITING COMPOUNDS IN THE PREPARATION OF PHARMACEUTICAL COMPOSITIONS INTENDED FOR THE TREATMENT OF CHONDRODYSTROPHIES, METHOD FOR IDENTIFYING INHIBITING COMPOUNDS, PHARMACEUTICAL COMPOSITIONS, CHONDRODYSTROPHY DIAGNOSIS METHOD AND APPLICATIONS THEREOF**

(30) Priority: 08.03.2007 ES 200700619
(71) Applicant: Consejo Superior De Investigaciones Científicas, 28006 Madrid (ES); Universidad Miguel Hernandez de Elche, 03202 Elche (Alicante) (ES)
(72) Inventor: NIETO TOLEDANO, María Angela, E-28006 Madrid (ES); ALVAREZ DE FRUTOS, Cristina, E-28006 Madrid (ES); VEGA, Sonia, E-28006 Madrid (ES)
(74) Representative: Pons Ariño, Angel
(86) International application number: PCT/ES2008/070042
(87) International publication number: WO 2008/107508

(57) **Abstract**

The invention relates to the use of snail1 activity inhibiting compounds in the preparation of pharmaceutical compositions intended for the treatment of chondrodystrophies, a method for identifying inhibiting compounds, pharmaceutical compositions, a chondrodystrophy diagnosis method and the applications thereof.

SEQUENCE LISTING

<110>  CONSEJO SUPERIOR DE INVESTIGACIONES CIENTÍFICAS
       UNIVERSIDAD MIGUEL HERNÁNDEZ

<120>  USE OF INHIBITORY COMPOUNDS OF SNAIL1 ACTIVITY IN THE PREPARATION OF

       PHARMACEUTICAL COMPOSITIONS USEFUL IN THE TREATMENT OF
CHONDRODYSPLASIAS,
       METHOD FOR IDENTIFYING SAID INHIBITORY COMPOUNDS, SAID
PHARMACEUTICAL
       COMPOSITIONS, METHOD FOR DIAGNOSING CHONDRODYSPLASIAS AND
APPLICATIONS THEREOF

<130>  SnailCondrod

<160>  21

<170>  PatentIn version 3.3

<210>  1
<211>  1613
<212>  DNA
<213>  Mouse


<220>
<221>  CDS
<222>  (64)..(858)
<223>  Mouse Snail1 coding sequence

<400>  1
cggagttgac taccgacctt gcgcgacccg gtgaccccga ctacctaggt cgctctggcc      60

aac atg ccg cgc tcc ttc ctg gtc agg aag ccg tcc gac ccc cgc cgg       108
    Met Pro Arg Ser Phe Leu Val Arg Lys Pro Ser Asp Pro Arg Arg
    1               5                   10                  15

aag ccc aac tat agc gag ctg cag gac gcg tgt gtg gag ttc acc ttc       156
Lys Pro Asn Tyr Ser Glu Leu Gln Asp Ala Cys Val Glu Phe Thr Phe
                20                  25                  30

cag cag ccc tac gac cag gcc cac ctg ctg gcc gcc atc cct ccg ccc       204
Gln Gln Pro Tyr Asp Gln Ala His Leu Leu Ala Ala Ile Pro Pro Pro
            35                  40                  45

gag gtc ctc aac ccc gcc gct tcg ctg ccc acc ctc atc tgg gac tct       252
Glu Val Leu Asn Pro Ala Ala Ser Leu Pro Thr Leu Ile Trp Asp Ser
        50                  55                  60

ctc ctg gta ccc caa gtg cgg ccg gtt gcc tgg gcc acc ctc ccg ctg       300
Leu Leu Val Pro Gln Val Arg Pro Val Ala Trp Ala Thr Leu Pro Leu
    65                  70                  75

cgg gag agc ccc aag gcc gta gag ctg acc tcg ctg tcc gat gag gac       348
Arg Glu Ser Pro Lys Ala Val Glu Leu Thr Ser Leu Ser Asp Glu Asp
80                  85                  90                  95

agt ggc aaa agc tcc cag ccg ccc agc ccg ccc tcg ccg gcg ccg tcg       396
Ser Gly Lys Ser Ser Gln Pro Pro Ser Pro Pro Ser Pro Ala Pro Ser
                100                 105                 110

tcc ttc tcg tcc acc tcg gcc tcg tcc ctg gag gcc gag gcc ttc atc       444
Ser Phe Ser Ser Thr Ser Ala Ser Ser Leu Glu Ala Glu Ala Phe Ile
            115                 120                 125

gcc ttc cct ggc ttg ggc caa ctt ccc aag cag ctg gcc agg ctc tcg       492
Ala Phe Pro Gly Leu Gly Gln Leu Pro Lys Gln Leu Ala Arg Leu Ser
        130                 135                 140

gtg gcc aag gac ccc cag tcg cgg aag atc ttc aac tgc aaa tat tgt     540
Val Ala Lys Asp Pro Gln Ser Arg Lys Ile Phe Asn Cys Lys Tyr Cys
    145                 150                 155

aac aag gag tac ctc agc ctg ggc gct ctg aag atg cac atc cga agc     588
Asn Lys Glu Tyr Leu Ser Leu Gly Ala Leu Lys Met His Ile Arg Ser
160                 165                 170                 175

cac acg ctg cct tgt gtc tgc acg acc tgt gga aag gcc ttc tct agg     636
His Thr Leu Pro Cys Val Cys Thr Thr Cys Gly Lys Ala Phe Ser Arg
                180                 185                 190

ccc tgg ctg ctt cag ggc cac gtc cgc acc cac act ggt gag aag cca     684
Pro Trp Leu Leu Gln Gly His Val Arg Thr His Thr Gly Glu Lys Pro
            195                 200                 205

ttc tcc tgc tcc cac tgc aac cgt gct ttt gct gac cgc tcc aac ctg     732
Phe Ser Cys Ser His Cys Asn Arg Ala Phe Ala Asp Arg Ser Asn Leu
        210                 215                 220

cgt gcc cac ctc caa acc cac tcg gat gtg aag aga tac cag tgc cag     780
Arg Ala His Leu Gln Thr His Ser Asp Val Lys Arg Tyr Gln Cys Gln
    225                 230                 235

gcc tgt gcc cga acc ttc tcc cgc atg tcc ttg ctc cac aag cac caa     828
Ala Cys Ala Arg Thr Phe Ser Arg Met Ser Leu Leu His Lys His Gln
240                 245                 250                 255

gag tct ggc tgc tcc gga ggc cct cgc tga ccctgctacc tccccatcct     878
Glu Ser Gly Cys Ser Gly Gly Pro Arg
                260

cgctggcatc ttcccggagc tcaccctcct cctcactgcc aggactcctt ccagccttgg     938

tccggggacc tgtggcgtcc atgtctggac ctggttcctg cttggctctc ttggtggcct     998

ttgccgcagg tggctgatgg agtgcctttg tacccgccca gagcctccta cccctcagta    1058

ttcatgaggt gtagcctctg gacacagctg cttcgagcca tagaactaaa gccaacccac    1118

tggctgggaa gcttgaaccc cgctcagggg accccacttc cctacctccc tcaaggaccc    1178

ttcaggccac cttctttgag gtacaacaga ctatgcaata gttcccctcc cccccacccc    1238

gtccagctgt aaccatgcct cagcagggtg gttactggac acatgtccag gtgcccctgg    1298

gcctgggcaa ctgtttcagc cccgcccccc atttgtcctg gtgacacctg tttcacagca    1358

gtttaactgt ctcagaaggg accatgaata atggccatca cttgttaggg gccaagtggg    1418

gtgcttcagc ctggccaatg tgtctcccag aactattttg gggcccaaca ggtggccccg    1478

ggagaaagat gtttacattt taaaggtatt tatattgtaa gcagcatttt gtatagttaa    1538

tatgtacagt ttattgatat tcaataaaat ggttaattta tatactaaaa aaaaaaaaaa    1598

aaaaaaaaaa aaaaa                                                     1613

<210>  2
<211>  264
<212>  PRT
<213>  Mouse

<400>  2

Met Pro Arg Ser Phe Leu Val Arg Lys Pro Ser Asp Pro Arg Arg Lys
1               5                   10                  15

Pro Asn Tyr Ser Glu Leu Gln Asp Ala Cys Val Glu Phe Thr Phe Gln
            20                  25                  30

Gln Pro Tyr Asp Gln Ala His Leu Leu Ala Ala Ile Pro Pro Pro Glu
        35                  40                  45

Val Leu Asn Pro Ala Ala Ser Leu Pro Thr Leu Ile Trp Asp Ser Leu
    50                  55                  60

Leu Val Pro Gln Val Arg Pro Val Ala Trp Ala Thr Leu Pro Leu Arg
65                  70                  75                  80

Glu Ser Pro Lys Ala Val Glu Leu Thr Ser Leu Ser Asp Glu Asp Ser
                85                  90                  95

Gly Lys Ser Ser Gln Pro Pro Ser Pro Pro Ser Pro Ala Pro Ser Ser
            100                 105                 110

Phe Ser Ser Thr Ser Ala Ser Ser Leu Glu Ala Glu Ala Phe Ile Ala
        115                 120                 125

Phe Pro Gly Leu Gly Gln Leu Pro Lys Gln Leu Ala Arg Leu Ser Val
    130                 135                 140

Ala Lys Asp Pro Gln Ser Arg Lys Ile Phe Asn Cys Lys Tyr Cys Asn
145                 150                 155                 160

Lys Glu Tyr Leu Ser Leu Gly Ala Leu Lys Met His Ile Arg Ser His
                165                 170                 175

Thr Leu Pro Cys Val Cys Thr Thr Cys Gly Lys Ala Phe Ser Arg Pro
            180                 185                 190

Trp Leu Leu Gln Gly His Val Arg Thr His Thr Gly Glu Lys Pro Phe
        195                 200                 205

Ser Cys Ser His Cys Asn Arg Ala Phe Ala Asp Arg Ser Asn Leu Arg
    210                 215                 220

Ala His Leu Gln Thr His Ser Asp Val Lys Arg Tyr Gln Cys Gln Ala
225                 230                 235                 240

Cys Ala Arg Thr Phe Ser Arg Met Ser Leu Leu His Lys His Gln Glu
                245                 250                 255

Ser Gly Cys Ser Gly Gly Pro Arg
            260

<210>  3
<211>  1708
<212>  DNA

<213>  Homo sapiens

<220>
<221>  CDS
<222>  (71)..(865)
<223>  Human Snail1 coding sequence

<400>  3
ggcacggcct agcgagtggt tcttctgcgc tactgctgcg cgaatcggcg accccagtgc      60

ctcgaccact atg ccg cgc tct ttc ctc gtc agg aag ccc tcc gac ccc      109
           Met Pro Arg Ser Phe Leu Val Arg Lys Pro Ser Asp Pro
           1               5                   10

aat cgg aag cct aac tac agc gag ctg cag gac tct aat cca gag ttt      157
Asn Arg Lys Pro Asn Tyr Ser Glu Leu Gln Asp Ser Asn Pro Glu Phe
    15                  20                  25

acc ttc cag cag ccc tac gac cag gcc cac ctg ctg gca gcc atc cca      205
Thr Phe Gln Gln Pro Tyr Asp Gln Ala His Leu Leu Ala Ala Ile Pro
30                  35                  40                  45

cct ccg gag atc ctc aac ccc acc gcc tcg ctg cca atg ctc atc tgg      253
Pro Pro Glu Ile Leu Asn Pro Thr Ala Ser Leu Pro Met Leu Ile Trp
                50                  55                  60

gac tct gtc ctg gcg ccc caa gcc cag cca att gcc tgg gcc tcc ctt      301
Asp Ser Val Leu Ala Pro Gln Ala Gln Pro Ile Ala Trp Ala Ser Leu
            65                  70                  75

cgg ctc cag gag agt ccc agg gtg gca gag ctg acc tcc ctg tca gat      349
Arg Leu Gln Glu Ser Pro Arg Val Ala Glu Leu Thr Ser Leu Ser Asp
        80                  85                  90

gag gac agt ggg aaa ggc tcc cag ccc ccc agc cca ccc tca ccg gct      397
Glu Asp Ser Gly Lys Gly Ser Gln Pro Pro Ser Pro Pro Ser Pro Ala
    95                  100                 105

cct tcg tcc ttc tcc tct act tca gtc tct tcc ttg gag gcc gag gcc      445
Pro Ser Ser Phe Ser Ser Thr Ser Val Ser Ser Leu Glu Ala Glu Ala
110                 115                 120                 125

tat gct gcc ttc cca ggc ttg ggc caa gtg ccc aag cag ctg gcc cag      493
Tyr Ala Ala Phe Pro Gly Leu Gly Gln Val Pro Lys Gln Leu Ala Gln
                130                 135                 140

ctc tct gag gcc aag gat ctc cag gct cga aag gcc ttc aac tgc aaa      541
Leu Ser Glu Ala Lys Asp Leu Gln Ala Arg Lys Ala Phe Asn Cys Lys
            145                 150                 155

tac tgc aac aag gaa tac ctc agc ctg ggt gcc ctc aag atg cac atc      589
Tyr Cys Asn Lys Glu Tyr Leu Ser Leu Gly Ala Leu Lys Met His Ile
        160                 165                 170

cga agc cac acg ctg ccc tgc gtc tgc gga acc tgc ggg aag gcc ttc      637
Arg Ser His Thr Leu Pro Cys Val Cys Gly Thr Cys Gly Lys Ala Phe
    175                 180                 185

tct agg ccc tgg ctg cta caa ggc cat gtc cgg acc cac act ggc gag      685
Ser Arg Pro Trp Leu Leu Gln Gly His Val Arg Thr His Thr Gly Glu
190                 195                 200                 205

aag ccc ttc tcc tgt ccc cac tgc agc cgt gcc ttc gct gac cgc tcc      733
Lys Pro Phe Ser Cys Pro His Cys Ser Arg Ala Phe Ala Asp Arg Ser
                210                 215                 220

aac ctg cgg gcc cac ctc cag acc cac tca gat gtc aag aag tac cag      781
Asn Leu Arg Ala His Leu Gln Thr His Ser Asp Val Lys Lys Tyr Gln

            225                 230                 235

tgc cag gcg tgt gct cgg acc ttc tcc cga atg tcc ctg ctc cac aag 829
Cys Gln Ala Cys Ala Arg Thr Phe Ser Arg Met Ser Leu Leu His Lys
        240                 245                 250

cac caa gag tcc ggc tgc tca gga tgt ccc cgc tga ccctcgaggc 875
His Gln Glu Ser Gly Cys Ser Gly Cys Pro Arg
    255                 260

tccctcttcc tctccatacc tgcccctgcc tgacagcctt ccccagctcc agcaggaagg 935

accccacatc cttctcactg ccatggaatt ccctcctgag tgccccactt ctggccacat 995

cagccccaca ggactttgat gaagaccatt ttctggttct gtgtcctctg cctgggctct 1055

ggaagaggcc ttcccatggc catttctgtg gagggagggc agctggcccc cagccctggg 1115

ggattcctga gctggcctgt ctgcgtgggt ttttgtatcc agagctgttt ggatacagct 1175

gctttgagct acaggacaaa ggctgacaga ctcactggga agctcccacc ccactcaggg 1235

gaccccactc ccctcacaca caccccccca caaggaaccc tcaggccacc ctccacgagg 1295

tgtgactaac tatgcaataa tccaccccca ggtgcagccc cagggcctgc ggaggcggtg 1355

gcagactaga gtctgagatg ccccgagccc aggcagctat ttcagcctcc tgtttggtgg 1415

ggtggcacct gtttcccggg caatttaaca atgtctgaaa agggactgtg agtaatggct 1475

gtcacttgtc gggggcccaa gtggggtgct ctggtctgac cgatgtgtct cccagaacta 1535

ttctgggggc ccgacaggtg ggcctgggag gaagatgttt acatttttaa aggtacactg 1595

gtatttatat ttcaaacatt ttgtatcaag gaaacgtttt gtatagttat atgtacagtt 1655

tattgatatt caataaagca gttaatttat atattaaaaa aaaaaaaaaa aaa 1708

<210> 4
<211> 264
<212> PRT
<213> Homo sapiens

<400> 4

Met Pro Arg Ser Phe Leu Val Arg Lys Pro Ser Asp Pro Asn Arg Lys
1               5                   10                  15

Pro Asn Tyr Ser Glu Leu Gln Asp Ser Asn Pro Glu Phe Thr Phe Gln
            20                  25                  30

Gln Pro Tyr Asp Gln Ala His Leu Leu Ala Ala Ile Pro Pro Pro Glu
        35                  40                  45

Ile Leu Asn Pro Thr Ala Ser Leu Pro Met Leu Ile Trp Asp Ser Val
    50                  55                  60

Leu Ala Pro Gln Ala Gln Pro Ile Ala Trp Ala Ser Leu Arg Leu Gln
65                  70                  75                  80

Glu Ser Pro Arg Val Ala Glu Leu Thr Ser Leu Ser Asp Glu Asp Ser
                85                  90                  95

Gly Lys Gly Ser Gln Pro Pro Ser Pro Pro Ser Pro Ala Pro Ser Ser
            100                 105                 110

Phe Ser Ser Thr Ser Val Ser Ser Leu Glu Ala Glu Ala Tyr Ala Ala
        115                 120                 125

Phe Pro Gly Leu Gly Gln Val Pro Lys Gln Leu Ala Gln Leu Ser Glu
    130                 135                 140

Ala Lys Asp Leu Gln Ala Arg Lys Ala Phe Asn Cys Lys Tyr Cys Asn
145                 150                 155                 160

Lys Glu Tyr Leu Ser Leu Gly Ala Leu Lys Met His Ile Arg Ser His
                165                 170                 175

Thr Leu Pro Cys Val Cys Gly Thr Cys Gly Lys Ala Phe Ser Arg Pro
            180                 185                 190

Trp Leu Leu Gln Gly His Val Arg Thr His Thr Gly Glu Lys Pro Phe
        195                 200                 205

Ser Cys Pro His Cys Ser Arg Ala Phe Ala Asp Arg Ser Asn Leu Arg
    210                 215                 220

Ala His Leu Gln Thr His Ser Asp Val Lys Lys Tyr Gln Cys Gln Ala
225                 230                 235                 240

Cys Ala Arg Thr Phe Ser Arg Met Ser Leu Leu His Lys His Gln Glu
                245                 250                 255

Ser Gly Cys Ser Gly Cys Pro Arg
            260

<210>  5
<211>  22
<212>  DNA
<213>  Artificial

<220>
<223>  Oligo_mGapdh_A

<400>  5
ctgagcaaga gaggccctat cc                                                22

<210>  6
<211>  20
<212>  DNA
<213>  Artificial

<220>
<223>  Oligo_mGapdh_B

<400>  6
ctccctaggc ccctcctgtt                                                   20

<210>  7
<211>  20
<212>  DNA
<213>  Artificial

<220>
<223>  Oligo_mP21_A

<400>  7
aggagccagg ccaagatggt                                                    20


<210>  8
<211>  22
<212>  DNA
<213>  Artificial

<220>
<223>  Oligo_mP21_B

<400>  8
gctttgacac ccacggtatt ca                                                 22


<210>  9
<211>  22
<212>  DNA
<213>  Artificial

<220>
<223>  Oligo_mSnail1_A

<400>  9
ccacactggt gagaagccat tc                                                 22


<210>  10
<211>  22
<212>  DNA
<213>  Artificial

<220>
<223>  Oligo_mSnail1_B

<400>  10
tcttcacatc cgagtgggtt tg                                                 22


<210>  11
<211>  25
<212>  RNA
<213>  Artificial

<220>
<223>  siRNA_I_A

<400>  11
cggaagaucu ucaacugcaa auauu                                              25


<210>  12
<211>  25
<212>  RNA
<213>  Artificial

<220>
<223>  siRNA_I_Bcomplem

<400>  12
aauauuugca guugaagauc uuccg                                            25


<210>  13
<211>  25
<212>  RNA
<213>  Artificial

<220>
<223>  siRNA_II_A

<400>  13
caaacccacu cggaugugaa gagau                                            25


<210>  14
<211>  25
<212>  RNA
<213>  Artificial

<220>
<223>  siRNA_II_Bcomplem

<400>  14
aucucuucac auccgagugg guuug                                            25


<210>  15
<211>  25
<212>  RNA
<213>  Artificial

<220>
<223>  siRNA_III_A

<400>  15
cagcugcuuc gagccauaga acuaa                                            25


<210>  16
<211>  25
<212>  RNA
<213>  Artificial

<220>
<223>  siRNA_III_Bcomplem

<400>  16
uuaguucuau ggcucgaagc agcug                                            25


<210>  17
<211>  19
<212>  DNA
<213>  Artificial

<220>
<223>  RNAm_snail

<400>  17
gatgcacatc cgaagccac                                                   19

## Description

This document discloses that the Snail1 gene transduces the signalling mediated by receptor FGFR3, which is responsible for chondrodysplasias (achondroplasia (ACH), thanatophoric dysplasia (TD) and hypochondroplasia (HCH)), it having been demonstrated that the aberrant activation of Snail1 is sufficient to reproduce a chondrodysplasia phenotype. Likewise, by means of interference RNA (siRNA) experiments on primary cultures, it was demonstrated that, when Snail1 function is prevented, FGFR3-mediated signalling in chondrocytes is prevented. This makes it possible to identify Snail1 as a therapeutic and diagnostic target for chondrodysplasia, as well as the use of the inhibitors thereof as drugs for the treatment of said disease.

### TECHNICAL FIELD OF THE INVENTION

This invention belongs in the field of biomedicine and, more specifically, the application of biotechnological tools for the diagnosis and treatment of human diseases, and, more specifically, of chondrodysplasias related to fibroblastic growth factor receptor 3 (achondrodysplasias (ACH), thanatophoric dysplasias (TD) and hypochondroplasias (HCH), hereinafter generically referred to as chondrodysplasias).

### STATE OF THE ART

The skeleton is formed by cartilages and bones. In turn, cartilages are formed by chondrocytes, whereas bones are formed by osteoblasts and osteoclasts. Chondrocytes and osteoblasts have a mesenchymal origin, whereas osteoclasts arise from the haematopoietic lineage.

Chondrocytes play an essential role in the formation of most skeletal components. In addition to their role during osteogenesis, the chondrocytes located in the growth plate control the longitudinal growth of bones. They gradually become differentiated inside the mesenchymal condensations, from proliferative chondrocytes until they reach their final differentiation stage as hypertrophic chondrocytes. This cell population gradually becomes surrounded by a calcified extracellular matrix, which favours the invasion of blood vessels from the perichondrium. This is when perichondrium cells begin to differentiate and become osteoblasts, forming the mineralised structure called bone collar (Karsenty and Wagner, 2002; Kronenberg, 2003).

After vascular invasion, hypertrophic chondrocytes die by apoptosis and osteoblasts begin to deposit the bone extracellular matrix, which is primarily composed of type I collagen. Chondrocytes are restricted to the growth plate, where, jointly with osteoblasts, they direct longitudinal bone growth..

In the growth plate, members of the FGF family and the receptors thereof, primarily receptor 3 (FGFR3), regulate the proliferation and differentiation of chondrocytes, inhibiting bone growth. FGFR3 is expressed in proliferative chondrocytes. Gain-of-function mutations of *Fgfr3* cause hypochondroplasia, achondroplasia, and thanatophoric dysplasia, the most severe variant of achondroplasia (Horton, 2006, and Huertz et al., 2006). Hypochondroplasia (HCH, OMIM 1460000) is the mildest form of this type of dwarfism and in 65% of cases is caused by mutation N540K in the tyrosine kinase 1 domain of the receptor (Bellus et al., 1995, and Prinos et al., 1995). Achondroplasia (ACH, OMIM 100800), which in 98% of cases is caused by mutation G380R in the transmembrane domain of the receptor, is the most common chondrodysplasia in humans (Rousseau et al., 1994; Shiang et al., 1994; Bellus et al., 1995b). Thanatophoric dysplasia presents a lethal phenotype and two varieties have been described by radiological diagnosis: type I and type II (TD I and TD II, OMIM 187600 and 187601, respectively; Tavormina et al., 1995). There exist murine models that reproduce the pathologies with mutations in the corresponding positions (Naski et al., 1998; Wang et al., 1999; Chen et al., 1999 and 2001; and Li et al., 1999). In all cases, the long-bone shortening phenotype is due to a disorganisation and shortening of the proliferative chondrocyte columns and to delayed differentiation. Defects in the proliferative chondrocytes are due to the activation of Stat1, which is responsible for the induction of cell cycle inhibitor p21 (Su et al., 1997; Sahni et al., 1999; Legeai-Mallet et al., 2004), and delayed differentiation is due to the activation of the MAPK signalling cascade (Murakami et al., 2004), which causes a reduction in the area of hypertrophic chondrocytes, in both animal and human models. On the contrary, the de-activation of *Fgfr3* in mice causes prolonged endochondral growth, resulting in a "long-bone" phenotype, which is accompanied by an expansion of the proliferative chondrocyte region in the growth plate (Deng et al., 1996, and Colvin et al., 1996). All these data give FGFR3 a significant role as a negative regulator of the proliferation of chondrocytes. This inhibition of proliferation by the FGF pathway is unique to chondrocytes (Wang et al., 2001) and is mediated by transcription factor STAT1, which increases the expression of cell cycle inhibitor p21, the final agent responsible for the interruption of the proliferation induced by this signalling pathway. Its levels may be considered to be a reflection of the activation of the FGFR3-mediated signalling pathway in the growth plate.

When mouse Snail was cloned (Nieto et al., 1992), it was observed that on day 12 in the embryonic development of the mouse, the predominant expression site of Snail was the pre-cartilage, including the pre-cartilages corresponding to the tail schlerotome, pre-vertebrae, ribs, limbs, and head. However, from day 14 in development, there is no expression of any of these sites in the pre-cartilage any more, except for the distal phalanges of the posterior extremities, which are the only sites where there is still pre-cartilage in the legs at this stage.

Snail1 has been recently, described as a direct repressor of type II collagen (Seki et al., 2003), which is characteristic of proliferative chondrocytes, and disappears when the latter cease to proliferate and differentiate into hypertrophic chondrocytes, the cell population that expresses type X collagen. In a completely independent context, it was observed that the presence of Snail attenuated the proliferation of epithelial cells in culture and evolved with an increase in p21 levels and an increase in the phosphorylation of ERK1 and ERK2 (Vega et al., 2004).

Summing up, human chondrodysplasias (which evolve with a delay and irregularity in cartilage formation) and the murine models generated to study them have been associated with mutations that generate a greater FGFR3 activity, which causes delayed differentiation and interruption of the proliferation of pathological chondrocyte populations, thereby preventing the individual's correct bone development.

There are several therapeutic approaches to these human chondrodysplasias. The surgical approach, which is very invasive and of long duration (Noonan et al., 1999): treatment with growth hormones (Hertel et al., 2005; Seino et al., 2000, and Ramaswami et al., 1999), which is not very effective in achondroplasia, aggravates the disproportion between the trunk and the extremities, and is very costly; and the use of natiuretic peptide CNP, which inhibits the FGF-mediated MAPK activation in the growth plate (Yasoda et al., 2004), but does not salvage the defects in the proliferation of chondrocytes.

Other strategies intend to decrease the receptor's tyrosine kinase activity with chemical agents or block binding of the ligand to FGFR3 with antibodies (Aveizer et al., 2003).

### DESCRIPTION OF THE INVENTION

### BRIEF DESCRIPTION

One object of this invention is a method for identifying a chondrodysplasia process, hereinafter method for identifying a chondrodysplasia process of the invention, based on the identification of the presence of Snail1 in a biological sample, which comprises the following steps:
a) identifying the presence of Snail1 in a biological sample of osseous origin, and
b) comparing the presence of Snail1 observed in a) to its absence in a control sample, where its presence is indicative of the existence of chondrodysplasia.

Another object of this invention is a method for identifying and evaluating the activity of inhibitory compounds of the Snail1 protein that are useful for the treatment of chondrodysplasia, hereinafter method for identifying compounds of this invention, which comprises the following steps:
a) placing a biological system with an expression of Snail1 that produces chondrodysplasia in contact with the candidate compound of this method, and incubation under suitable conditions,
b) determining a parameter that is indicative of the chondrodysplasia process, and
c) identifying a compound inhibitory of Snail1 protein activity when a reduction of said chondrodysplasia parameter is observed.

Another object of the invention is a biological system necessary for performing the method for identifying compounds of this invention, preferably a transgenic animal, preferably a mammal, and, most preferably, a non-human primate, where the expression of the Snail1 protein is inducible, in a constant or conditional manner, and where the expression thereof causes chondrodysplasia. A particular embodiment of said non-human mammalian animal of the invention is the transgenic mouse developed in the invention, the transgSnail1-ER mouse (Example 2).

Another object of this invention is the use of a compound or agent inhibitory of Snail1 protein activity, hereinafter use of an inhibitory compound of the invention, in the preparation of a drug or pharmaceutical composition useful for the treatment of a chondrodysplasia process, preferably human or veterinary.

Therefore, another object of the invention is the use of an inhibitory compound of Snail1 wherein the inhibitory compound is a nucleic acid or polynucleotide that prevents or reduces the expression of the gene that encodes the human Snail1 protein and which includes a nucleotide sequence selected from:
a) an anti-sense nucleotide sequence specific to the gene or mRNA sequence of the Snail1 protein,
b) a ribozyme specific to the mRNA of the Snail1 protein,
c) an aptamer specific to the mRNA of the Snail1 protein,
d) an interference RNA (siRNA or shRNA) specific,to the mRNA of the Snail1 protein, and
e) a microRNA (miRNA).specific to the Snail1 protein.

Another object of this invention is a pharmaceutical composition or a drug useful for the treatment of a chondrodysplasia process, hereinafter pharmaceutical composition of this invention, which comprises use of a therapeutically effective quantity of an inhibitory compound or agent of the Snail1 protein, jointly with, optionally, one or more pharmaceutically acceptable adjuvants and/or carriers.

Another object of this invention is the use of the pharmaceutical composition of the invention in a treatment method for a mammal, preferably a human being, affected by a chondrodysplasia process, hereinafter use of the pharmaceutical composition of this invention, which consists in administering said therapeutic composition that inhibits the chondrodysplasia process.

### DETAILED DESCRIPTION

This invention is based on the inventors' observing that the Snail1 gene transduces the signalling mediated by FGF receptor 3 (FGFR3), which is responsible for chondrodysplasia. In order to determine whether the Snail1 gene is responsible for this signalling pathway, its relative expression patterns were studied during embryonic development in mice, when the different processes wherein they are involved during the formation of mature bone in healthy mice take place (Example 1). The data show that the expression patterns coincide in embryonic stages, since the expression peak of Snail1 appears in the cell population that expresses FGFR3. These data are compatible with the fact that FGFR3 induces the expression of the Snail1 gene *in vivo.*

Also studied were the effects of the pathological activation of Snail1 on the bone development of transgenic mice with inducible expression of the Snail1 gene (Example 2). In this way, the presence of Snail1 has been directly related to the interruption of the proliferation of chondrocytes in transgenic mice wherein Snail1 was artificially activated (transgenic mouse of this invention, transgSnail1-ER mouse); at the same time, it was demonstrated that the aberrant activation of Snail1 is sufficient to reproduce a chondrodysplasia phenotype. The development and characterisation of this animal model, the transgSnail1-ER mouse, allows for the availability of animal models (this transgenesis process can be extrapolated to the development of other mammalian animals, including non-human primates, by those skilled in the art) wherein therapeutic compounds can be tested and characterised for use in the treatment of veterinary or human chondrodysplasia processes, which did not exist until now.

Thus, it was observed that Snail1 represses cell proliferation *in vivo,* which induces morphological defects in the growth plate in these bones (shorter bones). The changes described herein are reminiscent of those observed following experimental induction of chondrodysplasia in genetically modified mice that expressed mutated versions of FGFR3, which lead to the constitutive activation thereof (Chen et al., 1999 and 2001; Li et al., 1999; Wang et al., 1999).

Likewise, Snail1 has been associated with the FGFR3 signalling pathway by means of *in vitro* experiments in primary chondrocyte cultures obtained from mouse embryonic bones, which shows that the FGFR3-mediated signalling activates the expression of Snail1 (Example 3).

On the other hand, the activation of the Snail1 gene was studied in samples from patients with chondrodysplasia (Example 4). In this regard, in an unborn child with thanatophoric dysplasia, the mutation in FGFR3 responsible for this disease (severe form of chondrodysplasia with an always lethal evolution) was confirmed. It was observed that this mutation in FGFR3, which produces its constitutive activity, causes an aberrant activation of the Snail1 gene.

Finally, the interference RNA ("small interference", siRNA) experiments performed on primary cultures from mouse foetal bones showed that preventing the Snail1 function is sufficient to hinder FGFR3-mediated signalling in chondrocytes, reversing the interruption of the cell cycle (mediated by the expression of p21) and the activation of the MAPK pathway (recognised by the phosphorylation of ERK1/2), even in the presence of constitutively active FGFR3 (Example 3). Therefore, Snail1 is the mediator in FGFR3 signalling. The above-mentioned polynucleotides can be used in a gene therapy process wherein, using any technique or method, the integration thereof in the cells of a human patient is made possible.

If de-regulated Snail activity in embryonic cartilage is in itself capable of inducing achondroplasia, the presence of Snail1 in stages where it is normally repressed could be considered to be a marker of the achondroplasia phenotype of the type associated with FGFR3 - achondroplasia (ACH), thanatophoric dysplasia (TD) and hypochondroplasia (HCH) - and, therefore, its aberrant expression could be used as a diagnosis for any phenotype caused by the gain-of-function of FGFR3. On the other hand, if the activation of Snail1 is sufficient to induce the characteristics of the achondroplasias related to mutations that generate an increase in FGFR3 activity, i.e. there is a direct relation - and not only a temporary association - between Snail1 gene activity and the etiopathogeny of this disease, the inhibition thereof, e.g. the gene inhibition thereof, would become a form of anti-dysplasia therapy and Snail1 could be very useful in the identification of new anti-chondrodysplasia drugs. These therepeutic approaches are based on the use of inhibitory compounds or agents of said Snail1 protein. In fact, cases of chondrodysplasia of this type might appear even in the absence of mutations in FGFR3 by the sole pathological activation of Snail1 during the development of the cartilage-bone system.

Experiments concerning Snail1 expression in mouse embryos showed that there is a direct correlation between expression levels and the severity of the achondroplasia phenotype in mice, which indicates that Snail1 behaves as a risk factor and confirms its significance as a potential target for anti-achondroplasia therapies (Example 4).

The aberrant presence of Snail1 in embryonic cartilage (at a time when, under normal conditions, it no longer occurs) induces achondroplasia, regardless of the signal that has induced this pathological presence of Snail1. The inhibition of Snail1 by siRNA prevents the interruption of the proliferation of chondrocytes and the phosphorylation of ERKs even in the presence of constitutive FGFR3 activity. Therefore, the inhibition of Snail1 in the growth plate will prevent the effects of the gain-of-function of FGFR3 induced by any mechanism or the achondroplasias generated by the aberrant presence of Snail1 independent from FGFR3.

Therefore, one object of this invention is a method for identifying a chondrodysplasia process, hereinafter method for identifying a chondrodysplasia process of the invention, based on the identification of the presence of Snail1 in a biological sample, which comprises the following steps:
a) identifying the presence of Snail1 in a biological sample of osseous origin, and
b) comparing the presence of Snail1 observed in a) to its absence in a control sample, where its presence is indicative of the existence of chondrodysplasia.

As used in this invention, the term "chondrodysplasia process" refers to a disease with a chondrodysplasia phenotype, preferably human, wherein the biological action of Snail 1 is the cause of said disease, whether or not it is accompanied by an anomalous activation of FGFR3 - such as, for example: achondroplasia (ACH), thanatophoric dysplasia (TD) and hypochondroplasia (HCH) -. This identification of Snail1 in a biological sample of veterinary or medical origin from animals or human subjects may be extracted therefrom and, subsequently, the presence or absence of Snail1 therein identified *ex vivo,* which would be correlated with the diagnosis of a chondrodysplasia process in said subject and would allow for the definition and execution of a veterinary or medical therapeutic approach.

As used in this invention, the term "Snail1 gene" or "Snail1 protein" refers both to the Snail1 gene or protein, of different animal origin, preferably human (for example, mouse Snail1: SEQ ID NO 1 and NO 2, or human Snail1: SEQ ID NO 3 and NO 4, respectively), and to any nucleotide or amino acid (aa) sequence respectively analogous thereto. In the sense used in this description, the term "analogous" is intended to include any nucleotide or amino acid sequence that may be isolated or constructed on the basis of the nucleotide or amino acid sequences shown in this specification, for example, by the introduction of conservative or non-conservative nucleotide or amino acid substitutions, including the insertion of one or more nucleotides or amino acids, the addition of one or more nucleotides or amino acids at any of the ends of the molecule, or the deletion of one or more nucleotides or amino acids at any end or in the interior of the sequence, which is an encoding sequence or a peptide with an activity similar to the Snail1 sequence of the invention, i.e., capable of inducing chondrodysplasia.

In general, an analogous nucleotide or amino acid sequence is substantially homologous to the amino acid sequence previously discussed. In the sense used in this description, the expression "substantially homologous" means that the nucleotide or amino acid sequences in question have a degree of identity of, at least, 40%, preferably of, at least, 85%, or more preferably of, at least, 95%.

A particular object of the invention is the identification method for the invention wherein the identification of Snail1 of a) relates to the human form of Snail1 (hSnail1, whether the identification is in the form of a gene transcript (mRNA) or the protein form of the gene; SEQ ID NO 3 and 4). These analyses designed to identify Snail1 expression levels can be performed by the average person skilled in the field of biomedicine, thanks to the information disclosed in this invention and in the state of the art, by means of different techniques (Sambrook et al., 1989; Lambolez and Rossier, 2000; Egger et al., 2000; Folz and Nepluev, 2000, and Pfaffl, 2001).

Another particular object of the invention is the method for identifying a chondrodysplasia process wherein the identification of Snail1 is performed using specific Snail1 protein antibodies, preferably hSnail1 (Franci et al., 2006). The antibodies can be monoclonal or polyclonal.

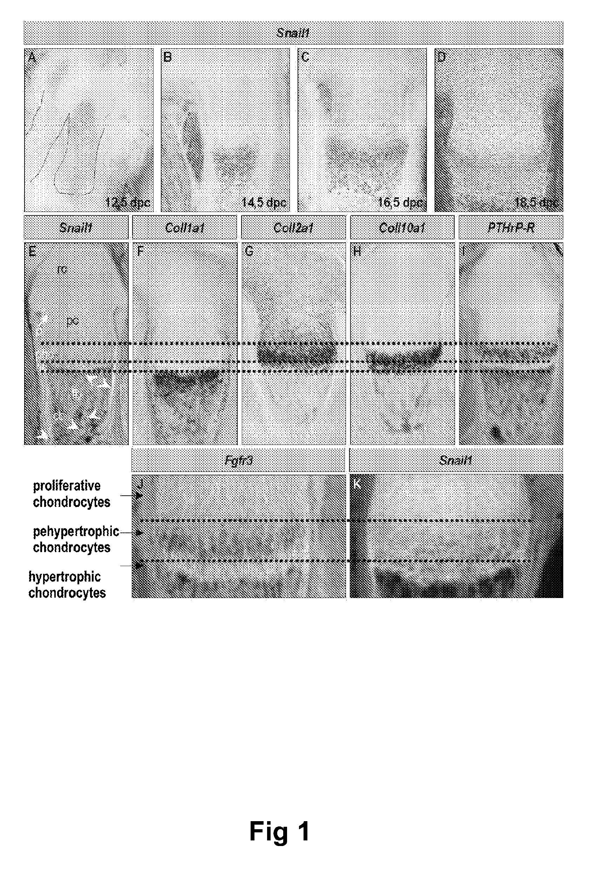

Another particular object of the invention is the method for identifying a chondrodysplasia process wherein the identification of Snail1 is performed by *in situ* hybridisation with a Snail1 precursor (see Figure 1).

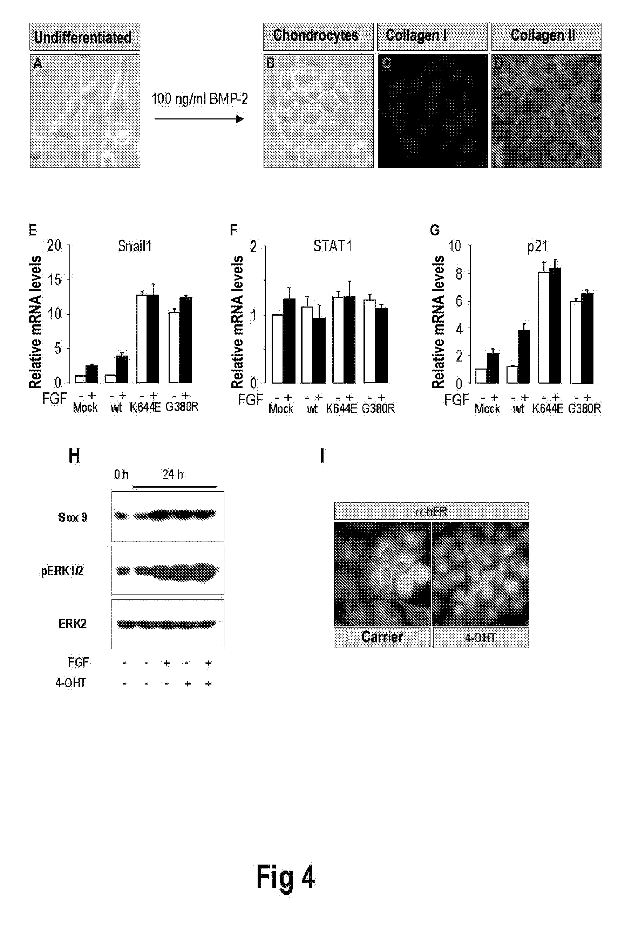

Another particular object of the invention is the method for identifying a chondrodysplasia process wherein the identification of Snail1 is performed by RT-PCR of a Snail1 gene precursor (Example 3, Figure 4). This method is based on the extraction of polyA+ RNA from a biological sample of osseous origin and a control tissue, and amplification of the Snail1-encoding sequence with suitable primer oligonucleotides (Boutet et al., 2006).

On the other hand, this diagnostic method for chondrodysplasia can be performed using Snail1 as the sole marker or jointly with other chondrodysplasia markers; for example, as a part of a biological expression microarray, either in gene form - from mRNA - or in protein form.

Another object of this invention is a method for identifying and evaluating the activity of inhibitory compounds of the Snail1 protein useful for the treatment of chondrodysplasia, hereinafter method for identifying compounds of this invention, which comprises the following steps:
a) placing a biological system with an expression of Snail1 that produces chondrodysplasia in contact with the candidate compound of this method, and incubation under suitable conditions,
b) determining a parameter that is indicative of the chondrodysplasia process, and
c) identifying a compound inhibitory of Snail1 protein activity when a reduction in said chondrodysplasia parameter is observed.

Another object of the invention is the method for identifying compounds of the invention where the biological system of point a) is a transgenic animal wherein the expression of the Snail1 protein is inducible, in a constant or conditional manner, and the expression thereof causes chondrodysplasia. A particular embodiment of the method for identifying compounds of this invention is one where the transgenic animal is the transgenic mouse of this invention (Example 2, transgSnail1-ER mouse).

Another object of the invention is a biological system necessary for performing the method for identifying compounds of this invention, preferably a transgenic animal, preferably a mammal, and, most preferably, a non-human primate, where the expression of the Snail1 protein is inducible, in a constant or conditional manner, and the expression thereof causes chondrodysplasia. A particular embodiment of said non-human mammalian animal of the invention is the transgenic mouse developed in the invention, the transgSnail1-ER mouse (Example 2).

Another object of this invention is the use of a compound or agent inhibitory of Snail1 protein activity, hereinafter use of an inhibitory compound of the invention, in the preparation of a drug or pharmaceutical composition useful for the treatment of a chondrodysplasia process, preferably human or veterinary.

As used in this invention, the term "inhibitory or antagonist compound/agent" refers to a molecule which, when it is bound to or interacts with the Snail1 protein (for example, SEQ ID NO 2, SEQ ID NO 4), or with functional fragments thereof, reduces or eliminates the intensity or the duration of the biological activity of said protein. This definition includes, furthermore, those compounds that prevent or reduce the expression of the Snail protein encoding gene (for example, SEQ ID NO 1, SEQ ID NO 3), i.e. that prevent or reduce gene transcription, mRNA maturation, mRNA translation and post-translational modification. An inhibitory agent may be composed of a peptide, a protein, a nucleic acid or polynucleotide, a carbohydrate, an antibody, a chemical compound or any other type of molecule that reduces or eliminates the effect and/or function of the Snail1 protein.

For illustrative purposes, said polynucleotide may be a polynucleotide that encodes a Snail1 protein gene or mRNA sequence specific anti-sense nucleotide sequence, or a polynucleotide that encodes a Snail1 protein mRNA specific ribozyme, or a polynucleotide that encodes a Snail1 protein mRNA specific aptamer, or a polynucleotide that encodes a Snail1 protein mRNA specific interference RNA ("small interference RNA" or siRNA, or an shRNA) or a microRNA (miRNA).

The above-mentioned polynucleotides can be used in a gene therapy process which, by means of any technique or procedure, allows for the integration thereof in the cells of a human patient. This objective may be achieved by administering a gene construct comprising one of the above-mentioned polynucleotides to these bone or cartilage cells in order to transform said cells, allowing for their expression in the interior thereof in such a way that expression of the Snail protein is inhibited. Advantageously, said gene construct can be included within a vector, such as, for example, an expression vector or a transfer vector.

As used in this invention, the term "vector" refers to systems used in the process of transferring an exogenous gene or an exogenous gene construct inside the cell, thereby allowing for the transport of exogenous genes and gene constructs. Said vectors may be non-viral or viral vectors (Pfeifer and Verma, 2001) and the administration thereof may be prepared by a person skilled in the art on the basis of the needs and specificities of each case.

Therefore, another object of the invention is the use of an inhibitory compound of Snail1 wherein the inhibitory compound is a nucleic acid or polynucleotide that prevents or reduces the expression of the gene that encodes the human Snail1 protein and includes a nucleotide sequence selected from:
a) an anti-sense nucleotide sequence specific to the gene or mRNA sequence of the Snail1 protein,
b) a ribozyme specific to the mRNA of the Snail1 protein,
c) an aptamer specific to the mRNA of the Snail1 protein,
d) an interference RNA (siRNA or shRNA) specific,to the mRNA of the Snail1 protein, and
e) a microRNA (miRNA).specific to the Snail1 protein.

Previously, antisense oligonucleotides have been disclosed and even patent protected (Patent US20060003956; Kajita et al., 2004); also disclosed and patent protected are siRNAs that inhibit the expression of Snail1 (Peinado et al., 2005; Tripathi et al., 2005). Any of these nucleotide sequences disclosed in the state of the art as inhibitory of the expression of Snail1 are incorporated as embodiments of this invention, as potentially useful therapeutic compounds for the preparation of drugs designed to treat a chondrodysplasia process. On the other hand, these gene inhibition techniques and, more specifically, transport of the compounds - antisense oligonucleotides, iRNA, ribozymes or aptamers - may be performed using liposomes, nanoparticles or other carriers that increase the success rate of said transfer to the interior of the cell, preferably the cell nucleus (Lu and Woodle, 2005; Hawker and Wooley, 2005). In principle, Snail1 mRNA translation inhibitors may be used which bind both to the encoding region and the non-encoding region, for example, in front of the 3' non-encoding area).

Thus, a particular embodiment of the invention is the use of an siRNA of d) wherein the iRNA preferably binds to the Snail mRNA gatgcacatccgaagccac (SEQ ID NO 17) fragment sequence or to another sequence that comprises the latter or a shorter fragment thereof (Patent US20060003956; the use of the siRNAs disclosed in this US patent are incorporated as particular embodiments within the scope of this patent).

Another particular embodiment of the invention is the use of an iRNA of d) wherein the siRNA is composed of a pair of nucleotide sequences, or a mixture thereof, belonging to the following group:
-I: 5'-CGG AAG AUC UUC AAC UGC AAA UAU U-3' (SEQ ID NO 11), complementary: 5'-AAU AUU UGC AGU UGA AGA UCU UCC G-3' (SEQ ID NO 12).
-II: 5'-CAA ACC CAC UCG GAU GUG AAG AGA U-3' (SEQ ID NO 13), complementary: 5'-AUC-UCU UCA CAU CCG AGU GGG UUU G-3' (SEQ ID NO 14), and
-III: 5'-CAG CUG CUU CGA GCC AUA GAA CUA A-3' (SEQ ID NO 15), complementary: 5'-UUA GUU CUA UGG CUC GAA GCA GCU G-3' (SEQ ID NO 16).

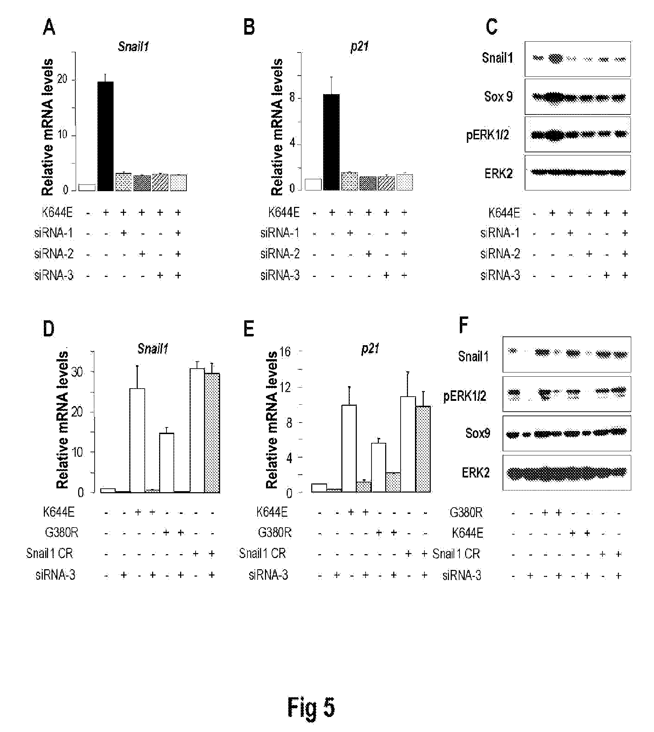

Pairs I and II bind to the encoding region of the Snail1 mRNA, whereas pair III binds to the 3' non-encoding region. The three pairs of siRNA sequences specified were active, and the image of the inhibition of Snail1 and p21 levels shown is representative of the results obtained by any of the three pairs of siRNAs (Figure 5), either by themselves or in combination.

Nucleotide sequences a)-e) mentioned above prevent gene expression in mRNA or mRNA expression in the Snail1 protein, and, therefore, destroy its biological function, and may be developed by a person skilled in the field of genetic engineering on the basis of the existing knowledge about transgenesis and gene expression destruction in the state of the art (Clarke, 2002; Patent US20020128220; Miyake et al., 2000; Puerta-Fernández et al., 2003; Kikuchi et al., 2003; Reynolds et al., 2004).

On the other hand, these compounds that inhibit the activity of Snail1 proteins may have a varied origin, such that they may be of natural origin (for example, vegetable, bacterial, viral or animal origin, or from eukaryotic microorganisms) or synthetic.

Another object of this invention is a pharmaceutical composition or a drug useful for the treatment of a chondrodysplasia process, hereinafter pharmaceutical composition of this invention, which comprises a therapeutically effective quantity of an inhibitory compound or agent of the Snail1 protein, jointly with, optionally, one or more pharmaceutically acceptable adjuvants and/or carriers.

A particular embodiment of this invention is a pharmaceutical composition wherein the inhibitory compound is a nucleic acid or polynucleotide which prevents or reduces the expression of the gene that encodes the human Snail1 protein and includes a nucleotide sequence selected from:
a) an anti-sense nucleotide sequence specific to the gene or mRNA sequence of the Snail1 protein,
b) a ribozyme specific to the mRNA of the Snail1 protein,
c) an aptamer specific to the mRNA of the Snail1 protein,
d) an interference RNA (iRNA) specific,to the mRNA of the Snail1 protein, and
e) a microRNA (miRNA).specific to the Snail1 protein.

Another particular embodiment of the invention is the pharmaceutical composition of the invention wherein the Snail1 inhibitor is an siRNA that preferably binds to the Snail mRNA gatgcacatccgaagccac (SEQ ID NO 17) fragment sequence or to another sequence that comprises the latter or a shorter fragment thereof (Patent US20060003956; the use of the siRNAs disclosed in this US patent are incorporated as particular embodiments within the scope of this patent).

Another particular embodiment of the invention is the use of the pharmaceutical composition of the invention wherein the Snail inhibitor is an iRNA composed of a pair of nucleotide sequences, or a mixture thereof, belonging to the following group:
-I: 5'-CGG AAG AUC UUC AAC UGC AAA UAU U-3' (SEQ ID NO 11), complementary: 5'-AAU AUU UGC AGU UGA AGA UCU UCC G-3' (SEQ ID NO 12).
-II: 5'-CAA ACC CAC UCG GAU GUG AAG AGA U-3' (SEQ ID NO 13), complementary: 5'-AUC UCU UCA CAU CCG AGU GGG UUU G-3' (SEQ ID NO 14), and
-III: 5'-CAG CUG CUU CGA GCC AUA GAA CUA A-3' (SEQ ID NO 15), complementary: 5'-UUA GUU CUA UGG CUC GAA GCA GCU G-3' (SEQ ID NO 16).

The pharmaceutically acceptable adjuvants and carriers that can be used in said compositions are the adjuvants and carriers known by those skilled in the art and usually used in the preparation of therapeutic compositions.

In the sense used in this description, the expression "therapeutically effective quantity" refers to the quantity of the inhibitory agent or compound of Snail1 protein activity calculated to produce the desired effect and, in general, will be determined, amongst other factors, by the compounds' characteristics, including the patient's age, condition, the severity of the alteration or disorder, and the administration route and frequency.

In a particular embodiment, said therapeutic composition is prepared in solid form or in aqueous suspension in a pharmaceutically acceptable diluent. The therapeutic composition provided by this invention may be administered by any suitable administration route; to this end, said composition will be formulated in the pharmaceutical form suitable for the chosen administration route. In a particular embodiment, administration of the therapeutic composition provided by this invention is performed by parenteral route, by oral route, by intraperitoneal route, by subcutaneous route, etc. A review of the different pharmaceutical forms to administer drugs and the necessary excipients to obtain them may be found, for example, in Faulí i Trillo, 1993.

Another object of this invention is the use of the pharmaceutical composition of the invention in a treatment method for a mammal, preferably a human being, suffering from a chondrodysplasia process, hereinafter use of the pharmaceutical composition of this invention, which consists in administering said therapeutic composition that inhibits the chondrodysplasia process.

Another particular object of this invention is the use of the pharmaceutical composition of this invention wherein the chondrodysplasia process caused by the biological action of Snail1 is accompanied by an anomalous activation of FGFR3, which, for illustrative purposes, and without this limiting the scope of the invention, belongs to the following group: achondroplasia (ACH), thanatophoric dysplasia (TD) and hypochondroplasia (HCH).

Finally, another particular object of the invention is the use of the pharmaceutical composition of this invention wherein the chondrodysplasia process caused by the biological action of Snail1 is not accompanied by anomalous FGFR3 activation.

### DESCRIPTION OF THE FIGURES

**Figure 1****. Snail1 is expressed during embryonic bone development in the populations involved in the longitudinal growth thereof.**
   Images of embryonic bone sections wherein the presence of mouse Snail1 mRNA has been detected by means of the *in situ* hybridisation technique. The upper panels (A-D) show the endogenous gene expression during development, first in the mesenchymal condensations and, subsequently, reduced to the hypertrophic condrocyte populations. The lower panels (E-I) compare their expression pattern with that of cell population marker molecules, and it may be observed that Snail1 is expressed at 18.5 days post-coitum (dpc) in the hypertrophic populations, the perichondrium and the osteoblasts. (J, K) detail of the growth plate of embryos at 18.5 dpc, which show the expression of Snail1 and FGFR3. In this and the following figures: wt, wild mouse; tg, transgenic mouse with inducible activation of Snail1; -TAM, without tamoxifen; +TAM, with tamoxifen.

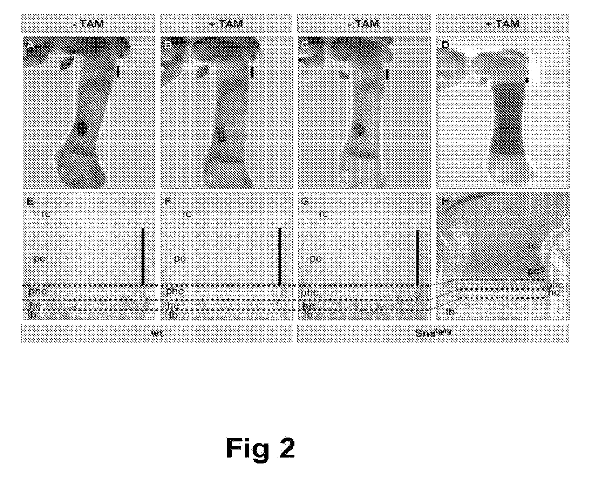

**Figure 2****. The long bones of embryos that express transgenic Snail1 are shorter.**
   (A-D) cartilage-bone staining that shows a reduction in the cartilage area (blue) at the expense of the populations of the growth plate in the bones of animals with activation of Snail1 induced by the administration of Tamoxifen. (E-H) histological sections which show that the shortening described above is at the expense of the proliferative chondrocyte populations.

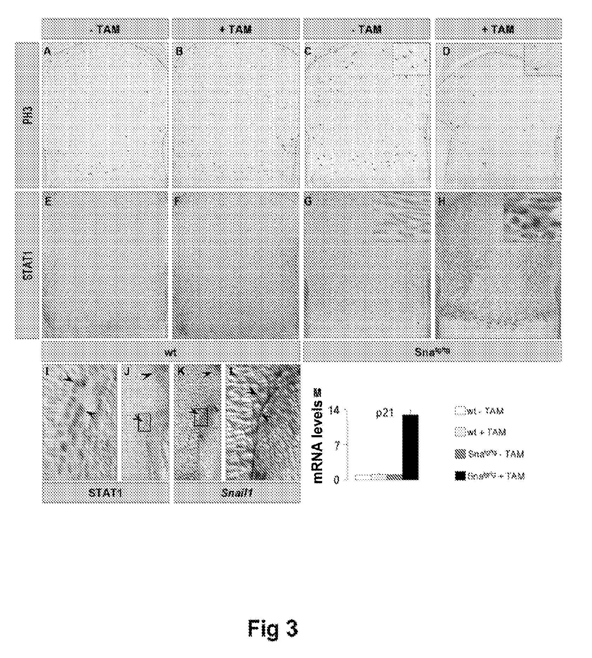

**Figure 3****. The presence of Snail1 in the growth plate inhibits cell proliferation.**
   (A-D) immunohistochemistry against PH3 to mark proliferating cells, where a drastic decrease in the number of proliferative cells may be observed in mice with an induced expression of Snail1. (E-H) in these same mice, the immunohistochemistry against STAT1 reveals an increase in the activation thereof (nuclear presence of the protein), which is accompanied by an increase in the levels of p21 mRNA (m). A detail of the endogenous co-expression of STAT1 and Snail1 under normal conditions in mouse bone can also be observed (I-L).
**Figure 4****. Snail1 is sufficient for FGFR3 signalling in the bone.**
   The activation of FGFR3 signalling in mouse primary chondrocyte cultures (A-D), due to the transfection of the mutated versions of FGFR3 representative of human chondrodysplasias ACH and TDII (K644E and G380R, respectively), induces the activation of Snail1, evaluated by the mRNA levels thereof (E), which is accompanied by the activation of p21 (G), without transcriptional variations of STAT1 (F). Moreover, the sole activation of Snail by the addition of 4-OHT in these cultures (I) causes the phosphorylation of ERK1/2 and, therefore, activation of the MAPK pathway, which is responsible for the delayed differentiation of chondrocytes, and may be measured by the persistence of Sox9 expression therein (H). Mock, primary cultures transfected with the empty vector; wt, primary cultures transfected with normal human FGFR3; K644E, primary cultures transfected with human FGFR3 with mutation K644E, responsible for most human thanatophoric dysplasias; G380R, primary cultures transfected with human FGFR3 with mutation G380R.
**Figure 5****. Snail1 is necessary for FGFR3 signalling in the bone.**
   The effects described in Figure 4 are prevented by treating said cultures with Snail1 siRNA.
**Figure 6****. There is a correlation between Snail1 expression levels and the severity of the achondroplasia phenotype in mice.**
   The phenotypes were divided into three groups, low (25%, A, B, G), medium (60%, C, D, H) and high (15%, E, F, I). The severity of the phenotype is related to the expression levels of Snail1.
**Figure 7****. The FGFR3 mutation responsible for thanatophoric dysplasia in humans activates the expression of Snail1.**
   Snail1 mRNA levels are increased in cartilage from a human thanatophoric foetus (severe form of achondroplasia related to the constitutive activation of FGFR3) with respect to one that has no skeletal development problems (a). The thanatophoric foetus exhibits the mutation described as constitutively active of said receptor (b). N, sample obtained from a foetus without bone problems; T, sample obtained from a foetus with thanatophoric dysplasia.

### EMBODIMENT EXAMPLES OF THE INVENTION

### Example 1. Snail1 is expressed during embryonic bone development of in the populations involved in the longitudinal growth thereof.

The presence of Snail1 mRNA in embryonic mouse bones was detected by means of the *in situ* hybridisation technique. The mouse embryos were obtained from the C57xCBA strain and their ages, established in days post-coitum (dpc), were determined by considering the day, where the vaginal plug is seen as day 0.5. The bones were desiccated at stages between 12.5 dpc and 18.5 dpc, respectively, and fixated in 4% paraformaldehyde in PBS/DEPC overnight. Subsequently, they were soaked in gelatin and cut with a vibratome, to obtain 50-m sections. The ISH in gelatin sections were performed as described in Blanco et al., 2002, using RNA probes labelled with DIG-11-UTP. Following hybridisation, the sections were processed as described in Cano et al., 2000.

Thus, it was shown that the endogenous expression of the Snail1 gene takes place during development; first in the mesenchymal condensations and, subsequently, reduced to the hypertrophic chondrocyte populations, the perichondrium, and the osteoblasts (Figure 1).

### Example 2. Snail1-ER transgenic mice present alterations in bone growth.

The pcDNA3-Snail1 plasmid, corresponding to the complete sequence of mouse *Snai*/*1* cDNA inserted in the pcDNA3 plasmid (Invitrogen; Cano et al., 2000), was used. pcDNA3-Snail-ER corresponds to the Snail1 encoding sequence bound to a mutated version of the binding domain to the human oestrogen receptor agonist that recognises the 4-OH-Tamoxifen synthetic ligand (Locascio et al., 2002).

The Snail1-ER transgene was designed as previously described (Locascio et al., 2002) and a transgenic mouse (transgSnail1-ER mouse) was generated for this construct in accordance with standard procedures (Hogan et al., 1994). For this study, an animal line was selected the transgenic protein expression of which was ubiquitous in the embryo. In this model, although the Snail1-ER protein is constitutively expressed, its function as a transcription factor only develops when the protein is translocated to the nucleus following treatment with tamoxifen (Danielian et al., 1998, and Feil et al., 1996 and 1997). The transgene is detected from the DNA taken from the animals' tail by PCR. The protein's sub-cellular location was analysed by immunohistochemistry using an anti-human oestrogen receptor antibody. The same antibody was used to evaluate the quantity of Snail1-ER protein in the different tissues obtained from the transgenic mice by Western Blot. The tamoxifen (Sigma) was first dissolved in ethanol (10% of the final volume) and subsequently in corn oil (Sigma) in order to obtain a final concentration of 30 mg/ml. Two consecutive intraperitioneal injections of tamoxifen are administered to pregnant females at days 12.5 and 14.5 dpc. The dose used was 57 g/g of weight of the female (Hayashi and McMahon, 2002).

Subsequently, in order to perform the cartilage-bone staining experiments, the desiccated embryos and the post-natal mice of the transgSnail1-ER transgenic mouse were fixated in 10% buffered formalin at ambient temperature for a minimum of 2 days. They were eviscerated and their skin removed. They were washed in water for between 2 hours and 2 days, depending on the size of the specimen. They were stained with an alcian blue solution (Sigma A5268) for 12-48 hours, in order to stain the cartilages. After washing the specimens in absolute Ethanol for at least two days, they were rehydrated and incubated in trypsin (Sigma T4799) until complete maceration of the tissue. They were transferred to an alizarin red S solution (Sigma A5533) in 0.5% KOH until the bones were stained red. The specimens were washed in consecutive solutions of 3:1, 1:1 and 1:3 0.5% KOH/glycerin solutions, and preserved in pure glycerin.

The study of the bones of transgSnail1-ER transgenic mice embryos showed that these were shorter than those that did not overexpress Snail1 (Figure 2), due to a reduction in the cartilage area at the expense of the proliferative chondrocyte populations. Furthermore, the expression of Snail1 was associated with an inhibition of the proliferation of proliferative chondrocytes and accompanied by the translocation of STAT1 to the nucleus and an increase in the expression of p21 (Figure 3).

In order to perform the immunohistochemistry assays, bone sections were analysed. In detail, the sections were obtained by means of cuts in microtomes and treated for 30 minutes in 1 mM EDTA at 100ºC, in order to unmask the antigen. After removing the paraffin residues and hydrating, they were permeabilised for 30 minutes in 0.5% Triton X-100 in PBS at ambient temperature and, subsequently, blocked for 1 hour in 0.1 % Tween, 10% FCS in PBS. The preparations were incubated with the primary antibodies in 0.1% Tween-20, 1% FCS in PBS for 16 hours at 4ºC, and with the secondary antibodies in 0.1% Tween-20, 1% FCS in PBS for 2 hours at ambient temperature. The preparations were mounted on Mowiol and preserved protected from light at 4ºC until they were viewed in a microscope.

The mRNA levels of p21 were analysed by RT-PCR. In this regard, and in the rest of the mRNA analyses of this invention, the quantitative PCR was performed using an ABI PRISM® 7000 quantitative PCR machine following the Syber-Green® method. The expression levels were calculated in accordance with the Ct method, using GAPDH as a normaliser and the wild mouse levels without any treatment or transfection as a calibrator. For these studies, the following primer sequences (all 5'-3') were used: *mGadph,* CTGAGCAAGAGAGGCCCTATCC (SEQ ID NO 5) and CTCCCTAGGCCCCTCCTGTT (SEQ ID NO 6); *mP21,* AGGAGCCAGGCCAAGATGGT (SEQ ID NO 7) and GCTTTGACACCCACGGTATTCA (SEQ ID NO 8); *mSnai*/*1,* CCACACTGGTGAGAAGCCATTC (SEQ ID NO 9) and TCTTCACATCCGAGTGGGTTTG (SEQ ID NO 10).

### Example 3. Snail1 is sufficient and necessary for FGFR3 signalling in chondrocytes.

The primary chondrocyte cultures were obtained from bone of the back legs of C57 animal embryos at 14.5 dpc, which were desiccated in culture medium ( -MEM, 1% BSA, 0.1% L-Glutamine, 0.1% penicillin/streptomycin). On the following day, they were trypsinised and treated with collagenase in DMEM and 10% serum. They were cultured in a medium (50% F-12, 50% DMEM, 10% FCS, 0.1% L-Glutamine, 0.1% penicillin/streptomycin) with a cell density of 1.5 x 10⁶ cells/P100 (Woods and Beier, 2006). After 5 days in culture, the differentiation was started with BMP-2 (Figure 4).

The activation of FGFR3 in mouse primary chondrocyte cultures with FGF induced the activation of Snail1, evaluated by means of the increase in the mRNA levels thereof (Figure 4E), which was accompanied by the activation of p21 (Figure 4G) and the activation of the MAPK signalling pathway; the phosphorylation levels of ERK1/2 and the levels of Sox9 were increased (Figure 4H), as measured by the Western Blot technique; moreover, the expression of the mutant form of FGFR3 causes the constitutive expression of Snail1, regardless of the presence of ligand (Figure 4E). The sole activation of Snail1 induced by the administration of 4-OHT in primary chondrocyte cultures from the transgenic mouse of this invention (transgSnail1-ER mouse) causes the activation of the MAPK signalling pathway, recognised by the increase in the phosphorylation levels of ERK1/2 and the levels of Sox9 (Figure 4H). As an example of a method to control the levels of Snail1 - and a model to treat a chondrodysplasia process -, a regulation assay of the expression of Snail1 was performed using specific siRNAs, and it was observed that said siRNAs prevented the expression of Snail1 and the increased expression of p21 and Sox9 and the phosphorylation of ERK1/2 (Figure 5). Three different siRNAs were used, two against encoding region sequences and one against the 3' non-encoding region, in order to ensure the specificity of the results. The sequences of these siRNAs were the following:
-I (encoding): 5'-CGG AAG AUC UUC AAC UGC AAA UAU U-3' (SEQ ID NO 11), complementary: 5'-AAU AUU UGC AGU UGA AGA UCU UCC G-3' (SEQ ID NO 12).
-II (encoding): 5-CAA ACC CAC UCG GAU GUG AAG AGA U-3' (SEQ ID NO 13), complementary: 5'-AUC-UCU UCA CAU CCG AGU GGG UUU G-3' (SEQ ID NO 14), and
-III (3' non-encoding area): 5'-CAG CUG CUU CGA GCC AUA GAA CUA A-3' (SEQ ID NO 15), complementary: 5'-UUA GUU CUA UGG CUC GAA GCA GCU G-3' (SEQ ID NO 16).

The three pairs of siRNA sequences specified were active, and the image of the inhibition of the levels of Snail1, p21, Sox9 and phosphorylation of ERK1/2 shown is representative of the results obtained by any of the three pairs of siRNAs (Figure 5), either by themselves or in combination.

### Example 4. There is a correlation between Snail1 expression levels and the severity of the achondroplasia phenotype in mice.

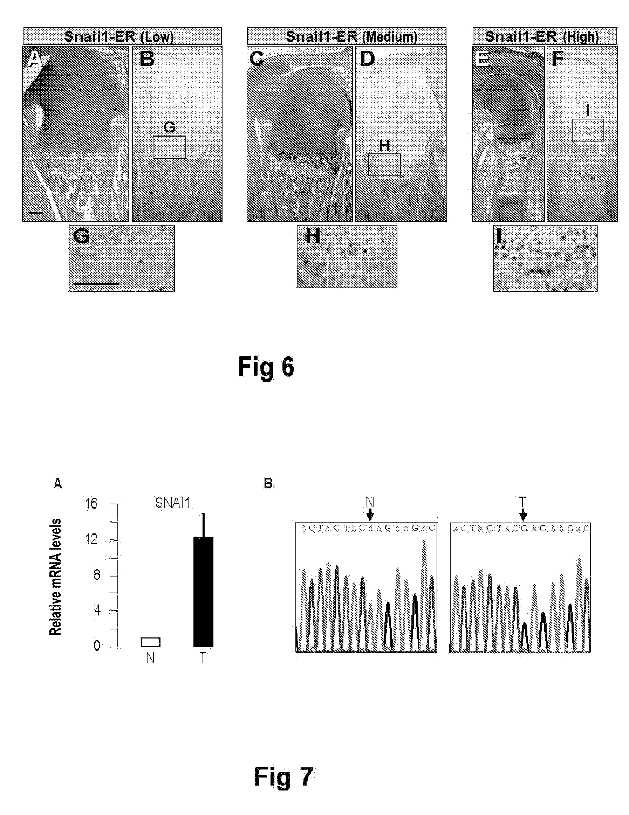

The bones of mouse embryos at 18.5 dpc were obtained as explained in Example 1. The sub-cellular location of the Snail1-ER fusion protein was analysed as explained in Example 2 and the histological staining of the samples was performed as specified in the section about materials and methods. It was observed that the greater the presence of active Snail1 protein in the bones (Figure 6F, I), the more aggressive the achondroplasia phenotype, with shorter, more curved bones (Figure 6E).

### Example 5. The mtuation of FGFR3 responsible for thanatophoric dysplasia in humans activates the expression of Snail1.

Snail1 mRNA levels are increased in cartilage from a human thanatophoric foetus (severe form of achondroplasia related to the constitutive activation of FGFR3) as opposed to one that has no skeletal development problems (Figure 7A). The thanatophoric foetus exhibits the mutation described as constitutively active of said receptor (Figure 7B).

### Materials and Methods

**Histological staining.** The sections were immersed in Haematoxylin (Harris Hematoxylin solution modified, Sigma HHS-16), for 1 minute, washed in tap water, and subsequently immersed in Eosin (Eosin Y Counterstain Solution, 0.5% Aqueous, Sigma HT110-2-32), for 30 seconds. After being washed in water, they were once again dehydrated, passed through Histoclear and mounted with Depex (BDH Laboratoires) in order to be analysed in the optical microscope.
**Western Blot analysis. Extraction of total proteins from the cell cultures.** The cells were washed with PBS and lysed with 150 I of RIPA buffer. The lysates were incubated for 30 minutes at 4ºC and centrifuged at 16,000 rpm for 10 minutes; it was assumed that the supernatant contained all the soluble proteins. The concentration of the extracts was measured by means of the Bradford method. The supernatants were mixed at 50% with 2X loading buffer and subject to electrophoresis in 8% agarose gel.
**Transfer**. The proteins were transferred to a nitrocellulose membrane in a mini-transblot cell at 110 V for 25 minutes. **Labelling with antibodies.** The membranes were blocked with 3% skim milk in TBS for 1 hour at ambient temperature and subsequently incubated with the corresponding primary antibodies diluted in 3% skim milk in TBS for 16 hours at 4ºC. After washing the membranes in TBS, they were incubated for 1 hour at ambient temperature with the corresponding peroxidase-coupled secondary antibodies. After washing the excess antibody, the membranes were developed by chemoluminescence and exposed on film. The total ERK-2 protein was used as a loading control.

## Claims

1. Method for identifying a chondrodysplasia process, **characterised in that** it is based on the identification of the aberrant presence of Snail1 in a biological sample, and which comprises at least one of the following steps:
a) identifying the presence of Snail1 in a biological sample of osseous origin from a mammal, and
b) comparing the presence of Snail1 observed in a) to its absence in a control sample, where its presence is indicative of the existence of chondrodysplasia.

2. Identification method, as claimed in claim 1, **characterised in that** the chondrodysplasia process is a disease with a dwarfism-type chondrodysplasia phenotype wherein the biological action of Snail1 is the cause of said disease, whether it is accompanied by an anomalous activation of receptor FGFR3 or not.

3. Identification method, as claimed in claim 2, **characterised in that** the disease belongs to the following group: achondroplasia (ACH), thanatophoric dysplasia (TD) or hypochondroplasia (HCH).

4. Identification method, as claimed in claim 1, **characterised in that** the identification of Snail1 refers to both the Snail1 gene and the protein, of different animal origin, preferably human.

5. Identification method, as claimed in claim 4, **characterised in that** Snail1 is the mouse Snail1 gene or protein with the sequence SEQ ID NO:1 and NO:2, respectively.

6. Identification method, as claimed in claim 4, **characterised in that** Snail1 is the human Snail1 gene or protein with the sequence SEQ ID NO:3 and NO:4, respectively.

7. Identification method, as claimed in claim 1, **characterised in that** the identification of Snail1 in a) refers to the human form of Snail1, whether the identification is performed in the form of a gene transcript (mRNA) or the protein form of the gene, with sequences SEQ ID NO:3 and 4.

8. Identification method, as claimed in claim 7, **characterised in that** the identification of Snail1 is performed using specific antibodies, either monoclonal or polyclonal, of the hSnail1 protein.

9. Identification method, as claimed in claim 7, **characterised in that** the identification of Snail1 is performed by means of *in situ* hybridisation with a Snail1 precursor.

10. Identification method, as claimed in claim 7, **characterised in that** the identification of Snail1 is performed by means of RT-PCR of a Snail1 precursor.

11. Method for identifying and evaluating the activity of inhibitory compounds of the Snail1 protein useful in the treatment of chondrodysplasia, **characterised in that** it comprises the following steps:
a) placing a biological system with an expression of Snail1 that produces chondrodysplasia in contact with the candidate compound of this method, and incubation under suitable conditions,
b) determining a parameter that is indicative of the chondrodysplasia process, and
c) identifying a compound inhibitory of Snail1 protein activity when a reduction of said chondrodysplasia parameter is observed.

12. Identification and evaluation method, as claimed in claim 11, **characterised in that** the biological system of step (a) is a non-human transgenic animal, preferably a mammal, and, more preferably, of a non-human primate, where the expression of the Snail1 protein is inducible, in a constant or conditional manner, and the expression thereof causes chondrodysplasia.

13. Identification and evaluation method, as claimed in claim 12, **characterised in that** the transgenic animal used is the transgSnail1-ER transgenic mouse.

14. Use of a biological system to perform the identification and evaluation method, as claimed in claims 11 to 13, **characterised in that** it is a non-human transgenic animal that may be induced to express the Snail1 protein, in a constant or conditional manner, and where the expression thereof causes chondrodysplasia.

15. Use of a biological system, as claimed in claim 14, **characterised in that** said non-human transgenic mammal animal is the transgSnail1-ER transgenic mouse.

16. Use of a compound or agent inhibitory of Snail1 protein activity in the preparation of a drug or pharmaceutical composition useful in the treatment of a chondrodysplasia process, preferably human or veterinary.

17. Use of a compound as claimed in claim 16, **characterised in that** it is a nucleic acid or polynucleotide that prevents or reduces the expression of the gene that encodes the human Snail1 protein and includes as nucleotide sequence selected from:
a) an anti-sense nucleotide sequence specific to the gene or mRNA sequence of the Snail1 protein,
b) a ribozyme specific to the mRNA of the Snail1 protein,
c) an aptamer specific to the mRNA of the Snail1 protein ,
d) an interference RNA (siRNA or shRNA) specific,to the mRNA of the Snail1 protein, and
e) a microRNA (miRNA).specific to the Snail1 protein.

18. Use of a compound, as claimed in claim 17, **characterised in that** the siRNA of d) is an iRNA that binds to the Snail mRNA sequence fragment gatgcacatccgaagccac (SEQ ID NO: 17) or to another sequence that comprises the latter or a shorter fragment thereof.

19. Use of a compound, as claimed in claim 17, **characterised in that** the iRNA of d) is a siRNA composed of a pair of nucleotide sequences, or a mixture thereof, belonging to the following group:
-I: 5'-CGG AAG AUC UUC AAC UGC AAA UAU U-3' (SEQ ID NO 11), complementary: 5'-AAU AUU UGC AGU UGA AGA UCU UCC G-3' (SEQ ID NO 12),
-II: 5'-CAA ACC CAC UCG GAU GUG AAG AGA U-3' (SEQ ID NO 13), complementary: 5'-AUC UCU UCA CAU CCG AGU GGG UUU G-3' (SEQ ID NO 14), and
-III: 5'-CAG CUG CUU CGA GCC AUA GAA CUA A-3' (SEQ ID NO 15) complementary: 5'-UUA GUU CUA UGG CUC GAA GCA GCU G-3' (SEQ ID NO 16).

20. Pharmaceutical composition or drug useful in the treatment of a chondrodysplasia process, **characterised in that** it comprises a therapeutically effective quantity of an inhibitory compound or agent of the Snail1 protein, jointly with, optionally, one or more pharmaceutically acceptable adjuvants and/or carriers.

21. Pharmaceutical composition, as claimed in claim 20, **characterised in that** the inhibitory compound is a nucleic acid or polynucleotide that prevents or reduces the expression of the gene that encodes the Snail1 protein and includes, at least, a nucleotide sequence selected from:
a) an anti-sense nucleotide sequence specific to the gene or mRNA sequence of the Snail1 protein,
b) a ribozyme specific to the mRNA ,of the Snail1 protein,
c) an aptamer specific to the mRNA of the Snail1 protein,
d) an interference RNA (siRNA or shRNA) specific,to the mRNA of the Snail1 protein, and
e) a microRNA (miRNA).specific to the Snail1 protein.

22. Pharmaceutical composition, as claimed in claim 21, **characterised in that** the siRNA of d) binds preferably to the Snail mRNA fragment sequence gatgcacatccgaagccac (SEQ ID NO:17) or to another sequence that comprises the latter or to a shorter fragment thereof.

23. Pharmaceutical composition, as claimed in claim 21, **characterised in that** the siRNA of d) is an iRNA composed of a pair of nucleotide sequences, or a mixture thereof, belonging to the following group:
-I: 5'-CGG AAG AUC UUC AAC UGC AAA UAU U-3' (SEQ ID NO 11), complementary: 5'-AAU AUU UGC AGU UGA AGA UCU UCC G-3' (SEQ ID NO 12),
-II: 5'-CAA ACC CAC UCG GAU GUG AAG AGA U-3' (SEQ ID NO 13), complementary: 5'-AUC UCU UCA CAU CCG AGU GGG UUU G-3' (SEQ ID NO 14), and
-III: 5'-CAG CUG CUU CGA GCC AUA GAA CUA A-3' (SEQ ID NO 15) complementary: 5'-UUA GUU CUA UGG CUC GAA GCA GCU G-3' (SEQ ID NO 16).

24. Use of the pharmaceutical composition, as claimed in claims 20 to 23, in a method for the treatment of a mammal, preferably a human being, affected by a chondrodysplasia process, consisting in the administration of said therapeutic compound that inhibits the chondrodysplasia process.

25. Use of the pharmaceutical composition, as claimed in claim 24, **characterised in that** the chondrodysplasia process caused by the biological action of Snail1 is accompanied by or equivalent to an anomalous activation of FGFR3, belonging to the following group: achondroplasia (ACH), thanatophoric dysplasia (TD) and hypochondroplasia (HCH).

26. Use of the pharmaceutical composition, as claimed in claim 24, **characterised in that** the chondrodysplasia caused by the biological action of Snail1 is not accompanied by anomalous activation of FGFR3.
